# EUROPÄISCHE PATENTSCHRIFT

(11) **EP 3 615 001 B1**
(45) Veröffentlichungstag und Bekanntmachung des Hinweises auf die Patenterteilung: **19.07.2023**
(21) Anmeldenummer: 18721013.3
(22) Anmeldetag: 24.04.2018
(51) Int. Cl.: A61K 9/00, A61K 47/10, A61K 47/42, A61L 27/34, A61L 27/52, A61P 41/00, A61L 27/22

(54) **ZELLFREIE KOMBINATION, HYDROGELARTIGES MATERIAL ODER HYDROGEL SOWIE VERWENDUNGEN DAVON**
CELL-FREE COMBINATION, HYDROGEL-LIKE MATERIAL OR HYDROGEL AND USES OF SAME
COMBINAISON EXEMPTE DE CELLULES, MATIÈRE DE TYPE HYDROGEL OU HYDROGEL AINSI QU'UTILISATIONS CORRESPONDANTES

(30) Priorität: 26.04.2017 DE 102017207049
(43) Veröffentlichungstag der Anmeldung: 04.03.2020
(73) Patentinhaber: TETEC Tissue Engineering Technologies AG, 72770 Reutlingen (DE)
(72) Erfinder: GAISSMAIER, Christoph, 72127 Kusterdingen (DE); CLAUSEN, Nils, 72076 Tübingen (DE); BENZ, Karin, 73037 Göppingen (DE)
(74) Vertreter: Patentanwälte Ruff, Wilhelm, Beier, Dauster & Partner mbB
(86) Internationale Anmeldenummer: PCT/EP2018/060486
(87) Internationale Veröffentlichungsnummer: WO 2018/197500

(56) Entgegenhaltungen:
- WO-A1-2018/041784
- DE-A1-102009 051 575

## Beschreibung

### ANWENDUNGSGEBIET UND STAND DER TECHNIK

Die vorliegende Erfindung betrifft eine zellfreie Kombination zur Anwendung bei der Wirkstofffreisetzung.

Es ist bekannt, Wirkstoffe über einen geeigneten Träger zu verabreichen. Bekannt ist zum Beispiel der Einsatz von Hormonpflastern, mit Opioiden ausgestatteten Schmerzpflastern, mit Hormonen beladenen Kunststoffimplantaten sowie mit Hormonen und/oder nicht-steroidalen Antiphlogistika versehenen Salben und Cremes. Nachteilig ist hierbei jedoch die Gefahr von Wirkstoffablösungen. Beispielsweise kann es im Falle von Hormon- oder Schmerzpflastern durch Schweiß, Bewegung oder Duschen zu Wirkstoffverlusten kommen. Je nach Wirkstoff können dabei Hautirritationen auftreten. In jedem Fall beinhalten vorzeitige Wirkstoffablösungen die Gefahr eines zu niedrigen Wirkstoffspiegels am gewünschten Wirkort.

In der Regel werden Wirkstoffe oral oder parenteral verabreicht und entfalten ihre Wirkung dadurch, dass eine wirksame Konzentration am gewünschten Wirkort im Körper eines Patienten erreicht wird. Um den Wirkstoffspiegel aufrechtzuerhalten, sind regelmäßige Wiederholungsdosen nötig, häufig täglich oder sogar mehrmals täglich.

Problematisch kann einerseits das Auftreten unerwünschter systemischer Wirkungen (Nebenwirkungen) sein, da häufig mehr oder weniger der gesamte Körper eines Patienten mit einem Wirkstoff geflutet werden muss, um auch einen ausreichenden Wirkstoffspiegel an einem oft nur lokal begrenzten Wirkort zu erzielen.

Ein weiteres Problem kann in einer systemischen Benachteiligung des gewünschten Wirkorts liegen. Eine derartige Benachteiligung kann sogar dazu führen, dass am Wirkort geringere Wirkstoffkonzentrationen auftreten als an anderen Orten/Kompartimenten eines Patientenkörpers, welche nicht Ziel der Behandlung sind.

Auch lokal verabreichte Wirkstoffe verbleiben oft zu kurz am gewünschten Wirkort und können mit relevanten Wirkstoffspiegeln in den restlichen Körper eines Patienten gelangen.

Die oben beschriebenen Nachteile sind besonders bei Komorbiditäten problematisch und können im Extremfall dazu führen, dass eine an sich erforderliche Therapie mit einem zur lokalen Wirkung beabsichtigten Wirkstoff aufgrund von Kontraindikationen wegen dessen systemischer Verteilung und Wirkung nicht durchgeführt werden kann.

Im Falle einer oralen Verabreichung stellt ein weiteres Problem häufig ein schwankender oder unzureichender Wirkstoffspiegel dar. Eine schwankende oder unzureichende Wirkstoffversorgung kann durch die mit einer oralen Verabreichung naturgemäß verknüpften Unterbrechungen wie Nachtpause, durch mangelnde Patienten-Compliance, durch individuelles Vergessen oder andere individuelle Faktoren, wie beispielsweise Resorptions- und/oder Verdauungsstörungen, bedingt sein.

Es ist weiterhin bekannt, Wirkstoffe in Form von hydrogelförmigen Formulierungen, insbesondere hydrogelförmigen Depotformulierungen, bereitzustellen. Die Verwendung von Hydrogelen bietet sich aufgrund ihrer besonderen Eigenschaften, wie beispielsweise ihres hohen Wassergehalts, ihrer Weichheit, ihrer Flexibilität sowie ihrer Biokompatibilität an. Viele Hydrogele bilden sich dabei erst nach einer Verabreichung in situ. Beispiele für derartige Hydrogele basieren auf Poloxameren und sind unter den Bezeichnungen Synperonics, Pluronics sowie Kolliphor kommerziell erhältlich. Diese Hydrogele bestehen aus nichtionischen Triblock-Copolymeren und sind aus einer zentralen hydrophoben Kette aus Polypropylenoxid zusammengesetzt, welche durch zwei hydrophile Polyethylenoxidketten flankiert ist.

Eine Depotformulierung ist unter der Bezeichnung MedinGel^{™} (BEPO^{™}) kommerziell erhältlich. Diese Formulierung besteht aus einem bioabbaubaren, mono-dispersen dreidimensionalen Netzwerk aus hydrophilen Polyethylenglykolketten, welche mit hydrophoben Polymilchsäureketten vernetzt sind. In einem wässrigen Medium formieren sich halbfeste Mikrodomänen, in welche verschiedene Wirkstoffe eingebracht werden können.

Eine weitere Depotformulierung stellt die Gamma- oder LiQuid-Polymer(LQP)-Technologie von InGel Labs (Groningen, Niederlande) dar. Die Formulierung basiert auf Copolymeren, welche Caprolacton- sowie Lactid-PEG-Blockeinheiten aufweisen, wobei die Formulierung ein flexibles und biokompatibles Depot für Wirkstoffe bildet. Die Technologie setzt einen Wirkstoff über einen Zeitraum von Tagen bis Wochen frei und basiert auf der Bildung eines thermo-reversiblen Hydrogels, welches einen Polymeranteil von 15 % bis 20 % aufweist. Der Rest des Hydrogels besteht aus Wasser und Wirkstoff.

Eine weitere Depotformulierung, die "stabilized injectable formulation" (SIF), von Foresee Pharmaceuticals Inc. (Newark, USA) befindet sich derzeit in klinischer Evaluierung. Die SIF-Technologie basiert auf bioabbaubaren Materialien in biokompatiblen Lösungsmitteln. Das System wird in "ready-to-use"-Fertigspritzen angeboten und bildet in situ ein Depot. Der Wirkstoff kann über einen Zeitraum von Tagen bis Monaten durch Diffusion und Biodegradation der Depotmatrix kontrolliert freigesetzt werden.

Nachteilig bei konventionellen Depotformulierungen ist, dass sie die einleitend beschriebenen Probleme nur teilweise lösen. Auch wiederholte Verabreichungen führen oft zu einem ungleichmäßigen Profil des Wirkstoffspiegels und sind vergleichsweise belastend oder aufwändig.

Ein weiterer Nachteil besteht darin, dass viele polymer- oder implantatbasierte Formulierungen eine Vielzahl von Herstellungsschritten erfordern, meistens unter Verwendung von organischen Lösungsmitteln. Deren Kompatibilität mit dem Wirkstoff ist oft schwer vorhersagbar und kompromittiert in der Regel die Stabilität oder Aktivität eines Wirkstoffs.

Aus der DE 10 2009 051 575 A1 ist die Verwendung einer biokompatiblen Zusammensetzung zur Inhibierung und/oder Prävention der Angiogenese oder Endothelzell-Proliferation bekannt. Die biokompatible Zusammensetzung ist zu einem Hydrogel bildenden Material polymerisierbar und basiert auf vernetzbarem Serumalbumin oder vernetzbarem Serumprotein.

### AUFGABE UND LÖSUNG

Der vorliegenden Erfindung liegt daher die Aufgabe zugrunde, ein Produkt bereitzustellen, welches die einleitend beschriebenen Nachteile wenigstens teilweise umgeht und insbesondere imstande ist, eine vorzugsweise kontinuierliche Wirkstofffreisetzung an einem Wirkort zu ermöglichen. Darüber hinaus soll sich das Produkt vorzugsweise durch eine gewisse Wirkstoffkompatibilität auszeichnen.

Diese Aufgabe wird erfindungsgemäß gelöst durch eine zellfreie Kombination gemäß unabhängigem Anspruch 1. Bevorzugte Ausgestaltungen der Erfindung sind in den abhängigen Ansprüchen definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung eine zellfreie Kombination zur Anwendung
- bei der kontrollierten, insbesondere verlangsamten und/oder oder verzögerten, Wirkstofffreisetzung.

Die zellfreie Kombination zeichnet sich dadurch aus, dass sie eine erste Komponente und eine zweite Komponente aufweist, wobei die erste Komponente vernetzbares Albumin und die zweite Komponente ein Vernetzungsmittel, insbesondere ein biokompatibles Vernetzungsmittel, für das Albumin enthält.

Unter dem Ausdruck "zellfreie Kombination" soll im Sinne der vorliegenden Erfindung eine Kombination verstanden werden, welche frei von Zellen, insbesondere frei von Gewebe- und/ oder Stammzellen, ist.

Unter dem Ausdruck "Kombination" soll im Sinne der vorliegenden Erfindung ein Produkt, insbesondere in Form eines Kits, Mehrkomponenten-Systems oder hydrogelbildenden Materials oder Hydrogels, verstanden werden, welches wenigstens zwei unterschiedliche Komponenten aufweist, wobei die Komponenten räumlich getrennt voneinander vorliegen oder miteinander vermischt sein können. Durch Mischen der Komponenten lässt sich vorzugsweise ein hydrogelbildendes Material oder ein Hydrogel herstellen, wobei die Herstellung des hydrogelbildenden Materials oder Hydrogels vorzugsweise auf einem Vernetzungsvorgang beruht.

Unter dem Ausdruck "vernetzbares Albumin" soll im Sinne der vorliegenden Erfindung ein Albumin verstanden werden, welches sich aufgrund seiner chemischen Beschaffenheit, insbesondere aufgrund einer geeigneten Funktionalisierung, mithilfe eines Vernetzungsmittels vernetzen lässt. Die Vernetzung kann dabei insbesondere auf der Ausbildung von ionischen Bindungen und/oder Wasserstoff-Brückenbindungen und/oder hydrophoben Wechselwirkungen und/oder kovalenten Bindungen zwischen dem Albumin und dem Vernetzungsmittel beruhen. Bevorzugt beruht die Vernetzung des Albumins auf der Ausbildung von kovalenten Bindungen zwischen dem Albumin und dem Vernetzungsmittel. Besonders bevorzugt beruht die Vernetzung des Albumins auf einer Michael-Addition zwischen elektrophilen Gruppen, bevorzugt Thiol-reaktive Gruppen, des Albumins und nukleophilen Gruppen, insbesondere Thiol- und/oder Thiolatgruppen, des Vernetzungsmittels.

Unter dem Ausdruck "Vernetzungsmittel für das Albumin" soll im Sinne der vorliegenden Erfindung ein Vernetzungsmittel verstanden werden, welches aufgrund seiner chemischen Beschaffenheit, insbesondere aufgrund einer geeigneten Funktionalisierung, in der Lage ist, das vernetzbare Albumin zu vernetzen. Die Vernetzung kann, wie im vorherigen Absatz erwähnt, insbesondere auf der Ausbildung von ionischen Bindungen und/oder Wasserstoff-Brückenbindungen und/oder hydrophoben Wechselwirkungen und/oder kovalenten Bindungen zwischen dem Albumin und dem Vernetzungsmittel beruhen. Bevorzugt beruht die Vernetzung des Albumins auf der Ausbildung von kovalenten Bindungen zwischen dem Albumin und dem Vernetzungsmittel. Besonders bevorzugt beruht die Vernetzung des Albumins auf einer Michael-Addition zwischen elektrophilen Gruppen, bevorzugt Thiol-reaktive Gruppen, des Albumins und nukleophilen Gruppen, insbesondere Thiol- und/oder Thiolatgruppen, des Vernetzungsmittels.

Unter dem Ausdruck "Michael-Akzeptor-Gruppen" sollen im Sinne der vorliegenden Erfindung elektrophile Gruppen verstanden werden, welche in der Lage sind, mit nukleophilen Gruppen (Michael-Donator-Gruppen) eine Michael-Addition einzugehen.

Unter dem Ausdruck "Michael-Donator-Gruppen" sollen im Sinne der vorliegenden Erfindung nukelophile Gruppen verstanden werden, welche in der Lage sind, mit elektrophilen Gruppen (Michael-Akzeptor-Gruppen) eine Michael-Addition einzugehen.

Unter dem Ausdruck "Thiol-reaktive Gruppen" sollen im Sinne der vorliegenden Erfindung funktionelle Gruppen verstanden werden, welche in der Lage sind, mit Thiol- und/oder Thiolatgruppen, vorzugsweise unter Ausbildung kovalenter Bindungen bzw. Verknüpfungen, zu reagieren.

Unter dem Ausdruck "Funktionalisierung" oder "funktionalisiert" bzw. "funktionalisieren" soll im Sinne der vorliegenden Erfindung im Zusammenhang des vernetzbaren Albumins jeder - abgeschlossene - Vorgang verstanden werden, mit welchem dem Albumin, beispielsweise durch Modifizierung mit Gruppen oder Hinzufügung von Gruppen, eine Funktion verliehen wird, welche das Albumin normalerweise nicht besitzt. Bezüglich geeigneter Gruppen sei auf die im Folgenden in Bezug auf eine Funktionalisierung des Albumins erwähnten Gruppen, wie insbesondere Maleimidgruppen, verwiesen.

Unter dem Ausdruck "Funktionalisierungsgrad" soll im Sinne der vorliegenden Erfindung im Zusammenhang des vernetzbaren Albumins die Anzahl von Gruppen, insbesondere elektrophile Gruppen, vorzugsweise Michael-Akzeptor-Gruppen wie insbesondere Maleimidgruppen, pro einzelnem Albuminmolekül verstanden werden.

Unter dem Ausdruck "thiolfunktionalisiert" soll im Sinne der vorliegenden Erfindung im Zusammenhang des Vernetzungsmittels ein Vernetzungsmittel verstanden werden, welches Thiolgruppen (SH-Gruppen) aufweist oder mit Thiolgruppen versehen ist.

Unter dem Ausdruck "thiolfunktionalisiert" soll im Sinne der vorliegenden Erfindung im Zusammenhang des Vernetzungsmittels ein Vernetzungsmittel verstanden werden, welches Thiolat-gruppen (S⁻-Gruppen) aufweist oder mit Thiolatgruppen versehen ist.

Unter dem Ausdruck "Funktionalisierungsgrad" soll im Sinne der vorliegenden Erfindung im Zusammenhang des Vernetzungsmittels die Anzahl von Gruppen, insbesondere nukelophilen Gruppen, vorzugsweise Michael-Donator-Gruppen wie insbesondere Thiolgruppen und/oder Thiolatgruppen, pro einzelnem Vernetzungsmittelmolekül verstanden werden. Im Falle eines polymeren Vernetzungsmittels, bei welchem nichtendständige Monomereinheiten (und optional auch endständige Monomereinheiten) funktionalisiert sind, bedeutet der Ausdruck "Funktionalisierungsgrad" im Sinne der vorliegenden Erfindung bevorzugt die Anzahl von Gruppen, insbesondere nukelophilen Gruppen, vorzugsweise Michael-Donator-Gruppen wie insbesondere Thiolgruppen und/oder Thiolatgruppen, pro 100 Monomereinheiten des polymeren Vernetzungsmittels.

Unter dem Ausdruck "Formulierung" soll im Sinne der vorliegenden Erfindung eine Darreichungsform oder Zubereitung, insbesondere in Form eines hydrogelbildenden Materials oder eines Hydrogels, verstanden werden, welche einen Wirkstoff, vorzugsweise einen pharmazeutischen Wirkstoff (Arzneistoff), enthält.

Unter dem Ausdruck "pharmazeutische Formulierung" soll im Sinne der vorliegenden Erfindung eine Darreichungsform oder Zubereitung, insbesondere in Form eines hydrogelbildenden Materials oder eines Hydrogels, verstanden werden, welche einen pharmazeutischen Wirkstoff (Arzneistoff), enthält.

Unter dem Ausdruck "Depotformulierung" soll im Sinne der vorliegenden Erfindung eine Formulierung verstanden werden, welche in der Lage ist, einen Wirkstoff über einen längeren Zeitraum, beispielsweise über einen Zeitraum von mehreren Tagen, Wochen oder Monaten, freizusetzen.

Unter dem Ausdruck "Wirkstoff" soll im Sinne der vorliegenden Erfindung eine Substanz bezeichnet werden, welche in einem Organismus eine spezifische Wirkung hat und/oder eine spezifische Reaktion hervorruft.

Unter dem Ausdruck "pharmazeutischer Wirkstoff" soll im Sinne der der vorliegenden Erfindung eine Substanz verstanden werden, welche eine Heilwirkung besitzt, d. h. als Arzneistoff wirkt.

Unter dem Ausdruck "Biopharmazeutikum" soll im Sinne der vorliegenden Erfindung ein Arzneistoff verstanden werden, welcher biotechnologisch oder mittels gentechnisch veränderten Organismen hergestellt ist.

Unter dem Ausdruck "Biopolymer" soll im Sinne der vorliegenden Erfindung ein Polymer verstanden werden, welches in der Natur vorkommt oder dessen Struktur mit einem in der Natur vorkommenden Polymer übereinstimmt.

Unter dem Ausdruck "hydrogelbildendes Material" soll im Sinne der vorliegenden Erfindung ein durch Vernetzen des Albumins mit dem Vernetzungsmittel hergestelltes oder herstellbares Material verstanden werden, welches zusammen mit Wasser oder einer wässrigen Flüssigkeit ein Hydrogel bildet.

Unter dem Ausdruck "Hydrogel" soll im Sinne der vorliegenden Erfindung ein wasserhaltiges Polymer-Netzwerk verstanden werden, welches ein hydrogelbildendes Material, vorzugsweise mit Vernetzungsmittel vernetztes Albumin, und Wasser aufweist.

Unter dem Ausdruck "Medizinprodukt" soll im Sinne der vorliegenden Erfindung ein Gegenstand oder Stoff verstanden werden, welcher zu medizinisch therapeutischen oder diagnostischen Zwecken für Menschen verwendet wird, wobei die bestimmungsgemäße Hauptwirkung im Unterschied zu Arzneimitteln primär nicht pharmakologisch, metabolisch oder immunologisch, sondern meist physikalisch oder physikochemisch erfolgt.

Völlig überraschend stellte sich heraus, dass eine zellfreie Kombination gemäß vorliegender Erfindung sowie insbesondere ein durch Mischen der Komponenten der zellfreien Kombination hergestelltes oder herstellbares hydrogelbildendes Material oder Hydrogel gemäß vorliegender Erfindung, bevorzugt als Depotmatrix oder Depotformulierung, zur kontrollierten Freisetzung von Wirkstoffen, insbesondere über mehrere Tage, ohne Beeinträchtigung der Struktur und/ oder der Funktion der Wirkstoffe verwendet werden kann. Auf diese Weise ist in vorteilhafter Weise insbesondere ein unterbrechungsfreier Wirkspiegel in vivo erzielbar.

Weiterhin stellte sich heraus, dass eine zellfreie Kombination gemäß vorliegender Erfindung sowie insbesondere ein durch Mischen der Komponenten der zellfreien Kombination hergestelltes oder herstellbares hydrogelbildendes Material oder Hydrogel gemäß vorliegender Erfindung, bevorzugt als Depotmatrix oder Depotformulierung, zur gleichmäßigen oder homogenen Freisetzung von Wirkstoffen, insbesondere über mehrere Tage, verwendet werden kann. Auf diese Weise kann mit besonderem Vorteil ein gleichmäßiger und insbesondere konstanter Wirkspiegel in vivo erzielt werden.

Weiterhin stellte sich heraus, dass die Herstellung eines hydrogelbildenden Materials oder eines Hydrogels gemäß vorliegender Erfindung durch Mischen der Komponenten der zellfreien Kombination keinen nachteiligen Einfluss auf die Struktur und/oder Aktivität von Wirkstoffen, welche in der ersten Komponente und/oder zweiten Komponente enthalten sein können, ausübt.

Mit besonderem Vorteil lässt sich insbesondere durch Veränderung von Funktionalisierungsgrad des vernetzbaren Albumins und/oder des Vernetzungsmittels und/oder durch Veränderung des vernetzbaren Albuminanteils in der ersten Komponente und/oder des Vernetzungsmittelanteils in der zweiten Komponente gezielt die Struktur, insbesondere der Vernetzungsgrad und/oder die Gelstärke, eines durch Mischen der Komponenten der zellfreien Kombination hergestelltes oder herstellbares hydrogelbildendes Material oder Hydrogel und mithin die Freisetzungsrate oder -geschwindigkeit von Wirkstoffen aus einem solchen Material oder Hydrogel bewusst steuern. Dadurch ist es insbesondere möglich, die Verweildauer und/oder die Konzentration von Wirkstoffen beispielsweise an einem gewünschten lokalen Wirkort gezielt zu beeinflussen, insbesondere zu erhöhen. Im Falle einer beabsichtigten systemischen Wirkung können über eine Beeinflussung der Freisetzungsrate etwaige unerwünschte Nebenwirkungen entweder gänzlich vermieden oder aber zumindest reduziert werden.

Die vorstehend beschriebenen Erkenntnisse konnten anhand von Freisetzungsuntersuchungen unter Verwendung von therapeutischen Antikörpern als Wirkstoffe erfolgreich verifiziert werden, worauf im Beispielteil noch näher eingegangen werden wird.

In Ausgestaltung der Erfindung ist die zellfreie Kombination für eine subkutane Verabreichung, vorzugsweise subkutane Injektion, hergerichtet. Mit anderen Worten wird die zellfreie Kombination gemäß einer Ausgestaltung der Erfindung für eine subkutane Verabreichung, vorzugsweise subkutane Injektion, verwendet. Durch eine subkutane Verabreichung ist es über einen Wirkstoff, welcher in der ersten Komponente und/oder zweiten Komponente der zellfreien Kombination enthalten sein kann, mit besonderem Vorteil möglich, einen lokalen und/oder systemischen Wirkstoffspiegel zu erzielen. So kann durch eine subkutane Verabreichung der zellfreien Kombination möglichst nahe an einen lokalen Wirkort ein ausschließlich oder nahezu ausschließlich lokal verweilender Wirkspiegel erzielt werden. Andererseits kann durch Diffusion des Wirkstoffs in Leitungsbahnen eines menschlichen Körpers, insbesondere in Blut- und/oder Lymphgefäße, eine systemische Wirkung erreicht werden.

In weiterer Ausgestaltung der Erfindung ist die zellfreie Kombination für eine chirurgische, insbesondere minimal-invasive, Verabreichung, vorzugsweise Injektion, insbesondere für eine Verabreichung, vorzugsweise Injektion, in eine Gewebehöhle, bevorzugt in eine durch Tumorentfernung entstandene Gewebehöhle, oder in ein Gelenk, vorzugsweise Kniegelenk, hergerichtet. Mit anderen Worten wird die zellfreie Kombination gemäß einer weiteren Ausgestaltung der Erfindung für eine chirurgische, insbesondere minimal-invasive, Verabreichung, vorzugsweise Injektion, insbesondere für eine Verabreichung, vorzugsweise Injektion, in eine Gewebehöhle, bevorzugt in eine durch Tumorentfernung entstandene Gewebehöhle, oder in ein Gelenk, vorzugsweise Kniegelenk, verwendet.

In weiterer Ausgestaltung der Erfindung ist die zellfreie Kombination für eine Facettengelenksinfiltration hergerichtet. Mit anderen Worten wird die zellfreie Kombination gemäß einer weiteren Ausgestaltung der Erfindung für eine Facettengelenksinfiltration verwendet.

Vorzugsweise handelt es sich bei dem vernetzbaren Albumin um vernetzbares humanes Albumin, insbesondere um vernetzbares humanes Serumalbumin. Dies trägt mit besonderem Vorteil zu einer weiteren Erhöhung der Wirkstoffkompatibilität der zellfreien Kombination sowie insbesondere eines durch Mischen der ersten Komponente und zweiten Komponente der zellfreien Kombination hergestellten oder herstellbaren hydrogelbildenden Materials oder Hydrogels bei.

Grundsätzlich kann das vernetzbare Albumin jedoch auch xenogenen, insbesondere bovinen, porcinen oder equinen, Ursprungs sein.

Bei dem Albumin handelt es sich um ein funktionalisiertes Albumin.

In weiterer Ausgestaltung der Erfindung ist das Albumin durch Gruppen, insbesondere elektrophile Gruppen, bevorzugt Michael-Akzeptor-Gruppen, besonders bevorzugt Thiol-reaktive Gruppen, funktionalisiert. Die Gruppen sind vorzugsweise ausgewählt aus der Gruppe bestehend aus Maleimidgruppen, Vinylsulfongruppen, Acrylatgruppen, Alkylhalogenidgruppen, Aziringruppen, Pyridylgruppen, Thionitrobenzolsäuregruppen, arylierende Gruppen und Kombinationen aus wenigstens zwei der genannten Gruppen.

Besonders bevorzugt ist das vernetzbare Albumin mit Maleimidgruppen funktionalisiert. Mit anderen Worten ist es erfindungsgemäß besonders bevorzugt, wenn es sich bei dem vernetzbaren Albumin um maleimidfunktionalisiertes Albumin, insbesondere maleimid-funktionalisiertes Serumalbumin, handelt. Insbesondere bevorzugt ist es, wenn es sich bei dem vernetzbaren Albumin um maleimidfunktionalisiertes Albumin, insbesondere maleimid-funktionalisiertes Serumalbumin, humanen Ursprungs handelt. Durch eine Funktionalisierung mit Maleimidgruppen kann eine besonders gute Vernetzung des Albumins und damit eine besonders kontrollierbare bzw. steuerbare Freisetzung von Wirkstoffen ermöglicht werden, welche in der ersten Komponente und/oder in der zweiten Komponente der zellfreien Kombination enthalten sein können.

Das vernetzbare Albumin weist einen Funktionalisierungsgrad von 5 Gruppen bis 35 Gruppen pro Albuminmolekül auf. Bezüglich geeigneter Gruppen sei auf die im vorherigen Absatz genannten Gruppen Bezug genommen. Bevorzugt handelt es sich bei den Gruppen um Maleimidgruppen. Über den Funktionalisierungsgrad des vernetzbaren Albumins lässt sich in besonders vorteilhafter Weise die Freisetzung von Wirkstoffen aus der zellfreien Kombination, insbesondere aus einem durch Mischen der ersten Komponente und zweiten Komponente der zellfreien Kombination hergestellten oder herstellbaren hydrogelbildenden Material oder Hydrogel steuern. So begünstigt ein höherer Funktionalisierungsgrad des Albumins mit besonderem Vorteil eine stärkere Vernetzung des Albumins und insbesondere die Ausbildung eines höheren Vernetzungsgrades der zellfreien Kombination, insbesondere eines durch Mischen der ersten Komponente und der zweiten Komponente der zellfreien Kombination hergestellten oder herstellbaren hydrogelbildenden Materials oder Hydrogels. Dadurch kann eine verlangsamte oder verzögerte Freisetzung von Wirkstoffen, welche in der ersten Komponente und/oder zweiten Komponente der zellfreien Kombination enthalten sein können, erreicht werden. Dies wiederum begünstigt mit besonderem Vorteil die Ausbildung eines längeren und insbesondere gleichmäßigeren Wirkstoffspiegels an einem gewünschten Wirkort. Ferner begünstigt eine geringere Wirkstofffreisetzung das Auftreten eines geringeren Wirkstoffspiegels, wodurch auf systemische Wirkungen beruhende Kontraindikationen vermieden oder aber wenigstens reduziert werden können.

Das vernetzbare Albumin weist einen Anteil von 0.1 Gew.-% bis 11 Gew.-% auf, bezogen auf das Gesamtvolumen der zellfreien Kombination, vorzugsweise bezogen auf das Gesamtvolumen einer in Form eines hydrogelbildenden Materials oder Hydrogels vorliegenden zellfreien Kombination. Der Albuminanteil der zellfreien Kombination stellt einen weiteren Parameter dar, mit welchem sich in vorteilhafter Weise die Freisetzung von Wirkstoffen, welche in der ersten Komponente und/oder zweiten Komponente der zellfreien Kombination enthalten sein können, beeinflussen lässt. So kann ein höherer Albuminanteil ebenfalls eine stärkere Vernetzung des Albumins und insbesondere die Ausbildung eines höheren Vernetzungsgrades der zellfreien Kombination, insbesondere eines durch Mischen der ersten Komponente und zweiten Komponente der zellfreien Kombination hergestellten oder herstellbaren hydrogelbildenden Materials oder Hydrogels, begünstigen. Dadurch kann eine verlangsamte oder verzögerte Freisetzung von Wirkstoffen, welche in der ersten Komponente und/oder zweiten Komponente der zellfreien Kombination enthalten sein können, erreicht werden. Dies wiederum begünstigt in vorteilhafter Weise die Ausbildung eines längeren und insbesondere gleichmäßigeren Wirkstoffspiegels an einem gewünschten Wirkort. Ferner begünstigt eine geringere Wirkstofffreisetzung das Auftreten eines geringeren Wirkstoffspiegels, wodurch unerwünschte systemische Wirkungen vermieden oder aber wenigstens reduziert werden können.

Ferner stellen Wechselwirkungen von Wirkstoffen, welche in der ersten Komponente und/oder zweiten Komponente der zellfreien Kombination enthalten sein können, mit dem Albumin, insbesondere ähnlich einer Plasmaproteinbindung, einen Faktor dar, welcher die Freisetzung von Wirkstoffen beeinflussen kann, so dass mit steigendem Albuminanteil auch eine zunehmende Kontrolle, insbesondere Verlangsamung oder Verzögerung, einer Wirkstofffreisetzung erzielt werden kann.

In weiterer Ausgestaltung der Erfindung weist das Vernetzungsmittel nukleophile Gruppen, insbesondere Michael-Donator-Gruppen, bevorzugt Thiolgruppen (SH-Gruppen) und/oder Thiolat-gruppen (S⁻-Gruppen), auf.

Das Vernetzungsmittel weist einen Funktionalisierungsgrad von wenigstens 2, insbesondere 2, 3 oder vier, auf. Ein polymeres Vernetzungsmittel, bei welchem nichtendständige (und optional endständige Monomereinheiten) funktionalisiert sind, kann beispielsweise einen Funktionalisierungsgrad von 1 % bis 25 % aufweisen, bezogen auf 100 Monomereinheiten des polymeren Vernetzungsmittels. Bezüglich geeigneter polymerer Vernetzungsmittel sei auf die nachfolgend gemachten Ausführungen, insbesondere auf die nachfolgend offenbarten thiolfunktionalisierten Polymere Bezug genommen. Der Funktionalisierungsgrad des Vernetzungsmittels stellt einen weiteren Parameter zur Beeinflussung der Freisetzung von Wirkstoffen, welche in der ersten Komponente und/oder zweiten Komponente der zellfreien Kombination enthalten sein können, dar.

Bei dem Vernetzungsmittel kann es sich weiterhin um ein lineares, d.h. unverzweigtes, Vernetzungsmittel handeln. Auch die Moleküllänge des Vernetzungsmittel stellt mit besonderem Vorteil einen Parameter dar, mittels welchem sich die Freisetzung von Wirkstoffen, welche in der ersten Komponente und/oder zweiten Komponente der zellfreien Kombination enthalten sein können, kontrollieren lässt. So können längerkettige Vernetzungsmittel zur Ausbildung von Strukturen, insbesondere in Form eines hydrogelbildenden Materials oder Hydrogels, mit größeren Zwischenräumen beitragen, so dass Wirkstoffe, welche in der ersten Komponente und/oder zweiten Komponente der zellfreien Kombination enthalten sein können, schneller und insbesondere über einen kürzeren Zeitraum freigesetzt werden können. Umgekehrt können kurzkettigere Vernetzungsmittel zu einer langsameren Wirkstofffreisetzung beitragen.

Bei dem Vernetzungsmittel kann es sich insbesondere um ein lineares Vernetzugsmittel mit zwei endständigen nukleophilen Gruppen, insbesondere Michael-Donator-Gruppen, bevorzugt Thiolgruppen (SH-Gruppen) und/oder Thiolatgruppen (S⁻-Gruppen), pro Vernetzungsmittelmolekül handeln.

Weiterhin kann es sich bei dem Vernetzungsmittel um ein verzweigtes, insbesondere mehrarmiges, beispielsweise drei- oder vierarmiges, Vernetzungsmittel handeln. Durch die Verwendung eines verzweigten Vernetzungsmittels kann mit besonderem Vorteil eine stärkere Vernetzung des Albumins und insbesondere die Ausbildung eines höheren Vernetzungsgrades der zellfreien Kombination, insbesondere eines durch Mischen der ersten Komponente und zweiten Komponente der zellfreien Kombination hergestellten oder herstellbaren hydrogelbildenden Materials oder Hydrogels, erzielt werden.

Insbesondere kann es sich bei dem Vernetzungsmittel um ein dreiarmiges Vernetzungsmittel mit drei endständigen nukleophilen Gruppen, insbesondere Michael-Donator-Gruppen, bevorzugt Thiolgruppen (SH-Gruppen) und/oder Thiolatgruppen (S⁻-Gruppen), pro Vernetzungsmittelmolekül oder um ein vierarmiges Vernetzungsmittel mit vier endständigen nukleophilen Gruppen, insbesondere Michael-Donator-Gruppen, bevorzugt Thiolgruppen (SH-Gruppen) und/oder Thiolatgruppen (S⁻-Gruppen), pro Vernetzungsmittelmolekül handeln.

Bei dem Vernetzungsmittel kann es sich weiterhin insbesondere um ein ein thiolfunktionalisiertes und/oder thiolatfunktionalisiertes Polymer, insbesondere Biopolymer, vorzugsweise Polysaccharid wie Mucopolysaccharid, handeln. Bezüglich geeigneter Polymere sei auf die im nachfolgenden Absatz offenbarten Polymere Bezug genommen.

Vorzugsweise ist das Vernetzungsmittel ausgewählt aus der Gruppe bestehend aus thiolfunktionalisiertes und/oder thiolatfunktionalisiertes (d.h. SH-funktionalisierte und/oder S⁻funktionalisierte) Polyethylenglykol wie Dithio-Polyethylenglykol bzw. Bis-Thio-Polyethylenglykol (Dithio-PEG bzw. Bis-Thio-PEG), thiolfunktionalisierte und/oder thiolatfunktionalisierte (d.h. SH-funktionalisierte und/oder S⁻-funktionalisierte) Hyaluronsäure, thiolfunktionalisierte und/oder thiolatfunktionalisierte (d.h. SH-funktionalisierte und/oder S⁻-funktionalisierte) Stärke, thiolfunktionalisiertes und/oder thiolatfunktionalisiertes (d.h. SH-funktionalisiertes und/oder S⁻-funktionalisiertes) Dextran, thiolfunktionalisierter und/oder thiolatfunktionalisierter (d.h. SH-funktionalisierter und/oder S⁻-funktionalisierter) Polyvinylalkohol, thiolfunktionalisiertes und/oder thiolatfunktionalisiertes (d.h. SH-funktionalisierter und/oder S⁻-funktionalisierter) Polyvinylpyrrolidon und Mischungen aus wenigstens zwei der genannten Vernetzungsmittel.

Besonders bevorzugt handelt es sich bei dem Vernetzungsmittel um Dithio-Polyethylenglykol bzw. Bis-Thio-Polyethylenglykol (Dithio-PEG bzw. Bis-Thio-PEG). Das Dithio-Polyethylenglykol kann insbesondere ein Molekulargewicht von 1000 Da bis 20000 Da aufweisen.

Weiterhin kann es sich bei dem Vernetzungsmittel um ein thiolfunktionalisiertes Polyethylenglykol mit drei endständigen Thiolgruppen, d.h. um ein dreiarmiges, thiolfunktionalisiertes Polyethylenglykol mit einem Funktionalisierungsgrad von 3, handeln. Vorzugsweise besitzt das thiolfunktionalisierte Polyethylenglykol ein Molekulargewicht von 1000 Da bis 30000 Da.

Weiterhin kann es sich bei dem Vernetzungsmittel um ein thiolfunktionalisiertes Polyethylenglykol mit vier endständigen Thiolgruppen, d.h. um ein vierarmiges, thiolfunktionalisiertes Polyethylenglykol mit einem Funktionalisierungsgrad von 4, handeln. Vorzugsweise besitzt das thiolfunktionalisierte Polyethylenglykol ein Molekulargewicht von 1000 Da bis 40000 Da.

Weiterhin kann es sich bei dem Vernetzungsmittel insbesondere um eine thiolfunktionalisierte Hyaluronsäure mit einem Molekulargewicht von 5000 Da bis 100000 Da handeln. Vorzugsweise weist die Hyaluronsäure in Bezug auf die Thiolgruppen einen Funktionalisierungsgrad von 1 % bis 25 % auf, bezogen auf 100 Monomereinheiten der Hyaluronsäure.

Das Vernetzungsmittel weist einen Anteil von 0.1 Gew.-% bis 30 Gew.-% auf, bezogen auf das Gesamtvolumen der zellfreien Kombination, vorzugsweise bezogen auf das Gesamtvolumen einer in Form eines hydrogelbildenden Materials oder Hydrogels vorliegenden zellfreien Kombination. Der Vernetzungsmittelanteil der zellfreien Kombination stellt einen weiteren Parameter dar, mit welchem sich in vorteilhafter Weise die Freisetzung von Wirkstoffen, welche in der ersten Komponente und/oder zweiten Komponente der zellfreien Kombination enthalten sein können, bewusst kontrollieren lässt. So lässt sich durch einen höheren Vernetzungsmittelanteil ebenfalls eine stärkere Vernetzung des Albumins und insbesondere die Ausbildung eines höheren Vernetzungsgrades der zellfreien Kombination, insbesondere eines durch Mischen der ersten Komponente und zweiten Komponente der zellfreien Kombination hergestellten oder herstellbaren hydrogelbildenden Materials oder Hydrogels, und mithin eine langsamere Wirkstofffreisetzung erzielen. Dies wiederum begünstigt mit besonderem Vorteil die Ausbildung eines längeren und insbesondere gleichmäßigeren Wirkstoffspiegels an einem gewünschten Wirkort. Ferner begünstigt eine geringere Wirkstofffreisetzung das Auftreten eines geringeren Wirkstoffspiegels, wodurch Nebenwirkungen vermieden oder aber wenigstens reduziert werden können.

Die erste Komponente, insbesondere nur die erste Komponente, enthält ferner einen Wirkstoff.

Die erste Komponente kann beispielsweise einen Anteil des Wirkstoffs von 0.1 Gew.-% bis 25 Gew.-% aufweisen, bezogen auf das Gesamtgewicht der ersten Komponente, insbesondere einer in wässriger Form vorliegenden ersten Komponente.

Alternativ oder in Kombination enthält die zweite Komponente, insbesondere nur die zweite Komponente, ferner einen Wirkstoff.

Die zweite Komponente kann beispielsweise einen Anteil des Wirkstoffs von 0.1 Gew.-% bis 25 Gew.-% aufweisen, bezogen auf das Gesamtgewicht der zweiten Komponente, insbesondere einer in wässriger Form vorliegenden zweiten Komponente.

In weiterer Ausgestaltung der Erfindung weist die erste Komponente und die zweite Komponente der zellfreien Kombination jeweils einen Wirkstoff auf.

In weiterer Ausgestaltung der Erfindung handelt es sich bei dem Wirkstoff um eine niedermolekulare Verbindung, d.h. um eine Verbindung mit einer Molekülmasse ≤ 800 g/mol. Bezüglich geeigneter niedermolekularer Verbindungen sei auf die nachfolgend im Zusammenhang des Wirkstoffs offenbarten niedermolekularen Verbindungen Bezug genommen.

In weiterer Ausgestaltung der Erfindung handelt es sich bei dem Wirkstoff um eine hochmolekulare Verbindung oder um ein Polymer, insbesondere Biopolymer. Bezüglich geeigneter Polymere, insbesondere Biopolymere, sei auf die nachfolgend im Zusammenhang des Wirkstoffs offenbarten Polymere verwiesen.

In weiterer Ausgestaltung der Erfindung handelt es sich bei dem Wirkstoff um einen Naturstoff, d.h. um einen von lebenden Organismen wie Pflanzen, Tieren oder Mikroorganismen produzierten Stoff.

In weiterer Ausgestaltung der Erfindung handelt es sich bei dem Wirkstoff um einen synthetisch hergestellten Wirkstoff.

In weiterer Ausgestaltung der Erfindung handelt es sich bei dem Wirkstoff um einen pharmazeutischen Wirkstoff (Arzneistoff), insbesondere um ein Biopharmazeutikum (Biologikum).

Der Wirkstoff ist ein Antikörper, insbesondere ein therapeutischer Antikörper, oder ein Antikörperfragment, insbesondere ein therapeutisches Antikörperfragment. Bei dem Antikörper kann es sich um einen monoklonalen Antikörper oder um einen polyklonalen Antikörper handeln. Bei dem Antikörperfragment handelt es sich um ein Fab-Fragment (Antigen-bindendes Fragment).

Der Antikörper ist bevorzugt ausgewählt aus der Gruppe bestehend aus Alemtuzumab, Apolizumab, Atezolizumab, Avelumab, Bevacizumab, Blinatumomab, Catumaxomab, Cetuximab, Daratumumab, Durvalumab, Eculizumab, Elotuzumab, Emicizumab, Epratuzumab, Gemtuzumab, Gemtuzumab-Ozogamicin, Ibritumomab-Tiuxetan, Inotuzumab-Ozogamicin, Ipilimumab, Mogamulizumab, Necitumumab, Nivolumab, Obinutuzumab, Ofatumumab, Olaratumab, Oregovomab, Panitumumab, Pembrolizumab, Pertuzumab, Ramucirumab, Rituximab, Rovalpituzumab-Tesirin, Siltuximab, Tremelimumab, Tositumomab, Trastuzumab, Zanolimumab, Adalimumab, Alefacept, Anifrolumab, Basiliximab, Belimumab, Brodalumab, Canakinumab, Certolizumab, Clazakizumab, Daclizumab, Fasinumab, Guselkumab, Golimumab, Infliximab, Ixekizumab, Mavrilimumab, Muromonab-CD3, Natalizumab, Secukinumab, Sifalimumab, Sirukumab, Tocilizumab, Ustekinumab, Vedolizumab, Alirocumab, Evolocumab, Abciximab, Bezlotoxumab, Motavizumab, Palivizumab, Aducanumab, Erenumab, Ocrelizumab, Solanezumab, Ranibizumab, Dupilumab, Efalizumab, Etanercept, Nemolizumab, Ustekinumab, Tositumab, Benralizumab, Mepolizumab, Omalizumab, Reslizumab, Tralokinumab, Denosumab, Romosozumab und Mischungen aus wenigstens zwei der genannten Antikörper.

Alternativ oder in Kombination handelt es sich bei dem Wirkstoff um ein Chondroprotektivum, welches vorzugsweise ausgewählt ist aus der Gruppe bestehend aus Hyaluronsäure, Chrondroitinsulfat, Glucosaminsulfat, Oxaceprol, Antioxidantien wie Vitamin E, Ademethionin (S-Adenosylmethionin SAM) und Mischungen aus wenigstens zwei der genannten Chondroprotektiva.

In weiterer Ausgestaltung der Erfindung weist die erste Komponente, insbesondere nur die erste Komponente, ferner Salze und/oder Aminosäuren und/oder Zuckeralkohole und/oder Saccharide auf. Bei den Salzen kann es sich insbesondere um Natriumchlorid und/oder Puffersalze, vorzugsweise um ein Phosphatpuffersalz, insbesondere um ein Gemisch aus Natriumdihydrogenphosphat und Dinatriumhydrogenphosphat, und/oder ein Kaliumphosphatsalz, insbesondere ein Gemisch aus Kaliumdihydrogenphosphat und Dikaliumhydrogenphosphat, handeln. Ein geeigneter Zuckeralkohol stellt beispielsweise Mannitol dar. Die Saccharide können ausgewählt sein aus der Gruppe bestehend aus Saccharose, Trehalose, Cyclodextrine und Mischungen davon

In weiterer Ausgestaltung der Erfindung weist die zweite Komponente, insbesondere nur die zweite Komponente, ferner Salze und/oder Aminosäuren und/oder Zuckeralkohole und/oder Saccharide auf. Bei den Salzen kann es sich insbesondere um Natriumchlorid und/oder Puffersalze, vorzugsweise um ein Phosphatpuffersalz, insbesondere um ein Gemisch aus Natriumdihydrogenphosphat und Dinatriumhydrogenphosphat, und/oder ein Kaliumphosphatsalz, insbesondere ein Gemisch aus Kaliumdihydrogenphosphat und Dikaliumhydrogenphosphat, handeln. Ein geeigneter Zuckeralkohol stellt beispielsweise Mannitol dar. Die Saccharide können ausgewählt sein aus der Gruppe bestehend aus Saccharose, Trehalose, Cyclodextrine und Mischungen davon

In weiterer Ausgestaltung der Erfindung weisen die erste Komponente und die zweite Komponente, ferner Salze und/oder Aminosäuren und/oder Zuckeralkohole und/oder Saccharide auf. Bezüglich in Frage kommender Salze und/oder Aminosäuren und/oder Zuckeralkohole und/oder Saccharide wird auf die in den vorherigen Absätzen gemachten Ausführungen Bezug genommen.

In weiterer Ausgestaltung der Erfindung liegen die erste Komponente und die zweite Komponente in einem Verhältnis, insbesondere Mischverhältnis, von erster Komponente zu zweiter Komponente von 10:1 bis 1:1, beispielsweise 4:1, vor.

In weiterer Ausgestaltung der Erfindung liegt die erste Komponente, insbesondere nur die erste Komponente, in wässriger Form, vorzugsweise in Form einer wässrigen Lösung, vor. Bevorzugt ist die wässrige Form, insbesondere wässrige Lösung, frei von organischen Lösungsmitteln. Dadurch kann die Wirkstoffkompatibilität der zellfreien Kombination und insbesondere eines durch Mischen der Komponenten der zellfreien Kombination hergestellten oder herstellbaren hydrogelbildenden Materials oder Hydrogels zusätzlich erhöht werden.

In weiterer Ausgestaltung der Erfindung liegt die zweite Komponente, insbesondere nur die zweite Komponente, in wässriger Form, vorzugsweise in Form einer wässrigen Lösung, vor. Bevorzugt ist die wässrige Form, insbesondere wässrige Lösung, frei von organischen Lösungsmitteln. Dadurch kann die Wirkstoffkompatibilität der zellfreien Kombination und insbesondere eines durch Mischen der Komponenten der zellfreien Kombination hergestellten oder herstellbaren hydrogelbildenden Materials oder Hydrogels zusätzlich erhöht werden.

In weiterer Ausgestaltung der Erfindung liegen die erste Komponente und die zweite Komponente jeweils in wässriger Form, vorzugsweise in Form einer wässrigen Lösung, vor. Bevorzugt ist die wässrige Form, insbesondere wässrige Lösung, frei von organischen Lösungsmitteln. Dadurch kann die Wirkstoffkompatibilität der zellfreien Kombination und insbesondere eines durch Mischen der Komponenten der zellfreien Kombination hergestellten oder herstellbaren hydrogelbildenden Materials oder Hydrogels in besonderer Weise erhöht werden.

In weiterer Ausgestaltung der Erfindung liegt die erste Komponente, insbesondere nur die erste Komponente, in Form eines Feststoffs, insbesondere in Form eines lyophilisierten Feststoffs, vor.

In weiterer Ausgestaltung der Erfindung liegt die zweite Komponente, insbesondere nur die zweite Komponente, in Form eines Feststoffs, insbesondere in Form eines lyophilisierten Feststoffs, vor.

In weiterer Ausgestaltung der Erfindung liegen die erste Komponente und die zweite Komponente jeweils in Form eines Feststoffs, insbesondere in Form eines lyophilisierten Feststoffs, vor.

In weiterer Ausgestaltung der Erfindung liegt die erste Komponente in Form eines Feststoffs, insbesondere in Form eines lyophilisierten Feststoffs, und die zweite Komponente in wässriger Form, vorzugsweise in Form einer wässrigen Lösung, vor. Bevorzugt ist die wässrige Form, insbesondere wässrige Lösung, frei von organischen Lösungsmitteln.

In weiterer Ausgestaltung der Erfindung liegt die erste Komponente in wässriger Form, vorzugsweise in Form einer wässrigen Lösung, und die zweite Komponente in Form eines Feststoffs, insbesondere in Form eines lyophilisierten Feststoffs, vor. Bevorzugt ist die wässrige Form, insbesondere wässrige Lösung, frei von organischen Lösungsmitteln.

In weiterer Ausgestaltung der Erfindung liegen die erste Komponente und die zweite Komponente räumlich getrennt voneinander vor. Vorzugsweise ist die zellfreie Kombination bei dieser Ausgestaltung der Erfindung als Kit oder Mehrkomponenten-System, insbesondere Zweikomponenten-System, ausgeführt. Zweckmäßigerweise sind hierzu die erste Komponente und die zweite Komponente in getrennten Behältnissen aufgenommen. Bei dem Kit oder Mehrkomponenten-System kann es sich insbesondere um ein medizinisches Kit oder medizinisches Mehrkomponenten-System, insbesondere Zweikomponenten-System, oder um ein pharmazeutisches Kit oder pharmazeutisches Mehrkomponenten-System, insbesondere Zweikomponenten-System, handeln.

In weiterer Ausgestaltung der Erfindung sind die erste Komponente und die zweite Komponente miteinander vermischt. Das vernetzbare Albumin ist dabei wenigstens teilweise, insbesondere nur teilweise oder vollständig, vernetzt. Bevorzugt liegt die zellfreie Kombination bei dieser Ausgestaltung der Erfindung wenigstens teilweise, insbesondere nur teilweise oder vollständig, als hydrogelbildendes Material oder Hydrogel, insbesondere als hydrogelförmige Formulierung, bevorzugt hydrogelförmige Depotformulierung, vor.

In weiterer Ausgestaltung der Erfindung handelt es sich bei der zellfreien Kombination um ein Medizinprodukt, insbesondere Implantat, oder um eine pharmazeutische Formulierung, insbesondere um eine pharmazeutische Depotformulierung.

Weitere Merkmale und Vorteile der Erfindung ergeben sich aus der nachfolgenden Beschreibung von bevorzugten Ausgestaltungen in Form von Beispielen. Dabei können einzelne Merkmale jeweils für sich alleine oder in Kombination miteinander verwirklicht sein. Die bevorzugten Ausgestaltungen dienen lediglich der weiteren Erläuterung und dem besseren Verständnis der Erfindung, ohne diese hierauf zu beschränken.

### BEISPIELTEIL

### 1. Herstellung mit Antikörper beladener Hydrogele

Komponente A:
enthaltend Antikörper 1 (AK1): Aus 0,525 mL einer Lösung aus maleimid-funktionalisiertem Albumin (44 mMol/L Maleimidgruppen, 2,4 mMol/L Albumin, Beladungsgrad 18,3 Maleimidfunktionen pro Albuminmolekül), 1 mL Hyaluronsäurelösung (20 mg/mL, MW 1,5 MDa) und 1,475 mL PBS wurde eine homogene Mischung hergestellt. Diese wurde mit 1000 µL der Antikörperlösung (144 kDa, 145 mg/mL) versetzt und wiederum homogen gemischt.
enthaltend Antikörper 2 (AK2): Aus 0,525 mL einer Lösung aus maleimid-funktionalisiertem Albumin (44 mMol/L Maleimidgruppen, 2,4 mMol/L Albumin, Beladungsgrad 18,3 Maleimidfunktionen pro Albuminmolekül), 1 mL Hyaluronsäurelösung (20 mg/mL, MW 1,5 MDa) und 1,475 mL PBS wurde eine homogene Mischung hergestellt. Diese wurde mit 1000 µL der Antikörperlösung (149 kDa, 115 mg/mL) versetzt und wiederum homogen gemischt.
enthaltend Antikörper 3 (Einzeldomänenantikörper, AK3): Aus 0,525 mL einer Lösung aus maleimid-funktionalisiertem Albumin (44 mMol/L Maleimidgruppen, 2,4 mMol/L Albumin, Beladungsgrad 18,3 Maleimidfunktionen pro Albuminmolekül), 1 mL Hyaluronsäurelösung (20 mg/mL, MW 1,5 MDa) und 1,475 mL PBS wurde eine homogene Mischung hergestellt. Diese wurde mit 1000 µL der Antikörperlösung (42 kDa, 38 mg/mL) versetzt und wiederum homogen gemischt.

### Komponente B:

Bisthio-Polyethylenglykol (MW 10 kDa) wurde in 0,9 %iger NaCl-Lösung, welche mit 10 %iger HCl auf pH 3-4 eingestellt war, zu einer Konzentration von 22,5 mMol/L Thiolgruppen gelöst. Die Thiolkonzentration wurde mittels Ellman-Assay bestimmt (verwendeter molarer Extinktionskoeffizient 14150).

### Hydrogel:

Für Freisetzungsuntersuchungen wurden die Komponenten A und B mit einer Doppelkolbenspritze mit aufgesetzter Mischvorrichtung im Verhältnis 4:1 (4 Teile Komponente A, 1 Teil Komponente) in situ in einer Edelstahlform zu zylindrischen Formkörpern von 1 mL Volumen (H - 5 mm, D - 16 mm) zu Hydrogelen vernetzt. Die Formkörper wurden nach 30 min aus der Form in die Freisetzungsapparatur überführt.

### 2. Freisetzungsuntersuchungen

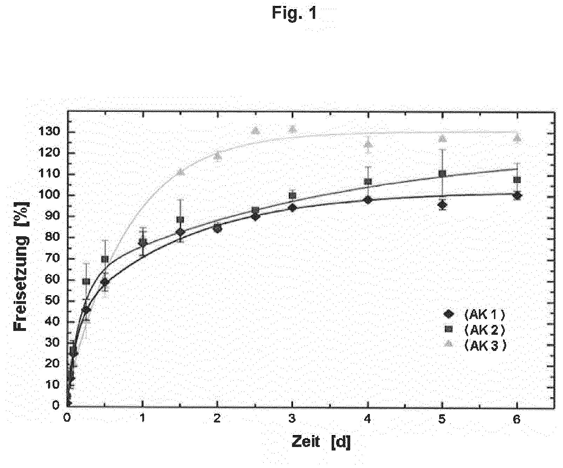

Die Hydrogel-Formkörper wurden in 100 mL PBS (50 mL bei AK3) in verschlossenen Glasflaschen bei 37 °C in einem Wasserbad inkubiert. Mittels eines Probennehmers wurden durch einen dicht durch den Flaschendeckel geführten Schlauch in Intervallen (ansteigend von 1 h bis 24 h) Proben von 1 mL genommen. Die Untersuchungsdauer bis zum letzten Messpunkt betrug 6 Tage. Der Gehalt an freigesetztem Antikörper in Lösung wurde mittels UV-Spektrophotometrie bei 280 nm unter Verwendung des entsprechenden molaren Extinktionskoeffizienten bestimmt. Die Freisetzungsprofile der AK1, AK 2 und AK 3 sind in Figur 1 dargestellt. Der Graph zeigt die deutlich verzögerte Freisetzung der Antikörper aus den Hydrogel-Formkörpern in die PBS-Lösung. Die Ordinate des Graphen wurde auf den Prozentsatz an insgesamt in die Lösung freigesetztem Antikörper im Verhältnis zur Gesamtmenge an im Hydrogel-Formkörper zum Zeitpunkt T0 vorhandenen Antikörper normiert.

## Patentansprüche

1. Zellfreie Kombination zur Anwendung
- bei der kontrollierten, insbesondere verlangsamten oder verzögerten, Wirkstofffreisetzung,
wobei
die zellfreie Kombination eine erste Komponente und eine zweite Komponente aufweist, wobei die erste Komponente vernetzbares Albumin und die zweite Komponente ein Vernetzungsmittel für das Albumin enthält, **dadurch gekennzeichnet, dass** das vernetzbare Albumin einen Funktionalisierungsgrad von 5 Gruppen bis 35 Gruppen pro Albuminmolekül aufweist, das Vernetzungsmittel einen Funktionalisierungsgrad von wenigstens zwei Gruppen pro einzelnem Vernetzungsmittelmolekül oder bei einem polymeren Vernetzungsmittel, bei welchem nichtendständige Monomereinheiten und optional auch endständige Monomereinheiten funktionalisiert sind, pro 100 Monomereinheiten des polymeren Vernetzungsmittels aufweist, das vernetzbare Albumin einen Anteil von 0.1 Gew.-% bis 11 Gew.-%, bezogen auf das Gesamtvolumen der zellfreien Kombination, und das Vernetzungsmittel einen Anteil von 0.1 Gew.-% bis 30 Gew.-%, bezogen auf das Gesamtvolumen der zellfreien Kombination, aufweist und die erste Komponente und/oder die zweite Komponente ferner einen Wirkstoff enthalten, wobei es sich bei dem Wirkstoff um einen Antikörper, um ein Fab-Fragment eines Antikörpers und/oder um ein Chondroprotektivum handelt.

2. Zellfreie Kombination nach Anspruch 1, **dadurch gekennzeichnet, dass** die Kombination für eine subkutane Verabreichung, vorzugsweise Injektion, hergerichtet ist oder verwendet wird.

3. Zellfreie Kombination nach Anspruch 1, **dadurch gekennzeichnet, dass** die Kombination für eine chirurgische, insbesondere minimal-invasive, Verabreichung, vorzugsweise Injektion, insbesondere in ein Gelenk, vorzugsweise Kniegelenk, oder in eine Gewebehöhle, insbesondere in eine durch Tumorentfernung entstandene Gewebehöhle, hergerichtet ist oder verwendet wird.

4. Zellfreie Kombination nach einem der vorhergehenden Ansprüche, **dadurch gekennzeichnet, dass** das Albumin mit Gruppen, insbesondere elektrophilen Gruppen, funktionalisiert ist, welche ausgewählt sind aus der Gruppe bestehend aus Maleimidgruppen, Vinylsulfongruppen, Acrylatgruppen, Alkylhalogenidgruppen, Aziringruppen, Pyridylgruppen, Thionitrobenzolsäuregruppen, arylierende Gruppen und Kombinationen aus wenigsten zwei der genannten Gruppen.

5. Zellfreie Kombination nach einem der vorhergehenden Ansprüche, **dadurch gekennzeichnet, dass** es sich bei dem vernetzbaren Albumin um maleimidfunktionalisiertes Albumin, insbesondere maleimidfunktionalisiertes Serumalbumin, vorzugsweise humanen Ursprungs, handelt.

6. Zellfreie Kombination nach einem der vorhergehenden Ansprüche, **dadurch gekennzeichnet, dass** das Vernetzungsmittel nukleophile Gruppen, bevorzugt Thiol- und/oder Thiolat-Gruppen, aufweist.

7. Zellfreie Kombination nach einem der vorhergehenden Ansprüche, **dadurch gekennzeichnet, dass** das Vernetzungsmittel ausgewählt ist aus der Gruppe bestehend aus thiolfunktionalisiertes und/oder thiolatfunktionalisiertes Polyethylenglykol, wie Dithio-Polyethylenglykol, thiolfunktionalisierte und/oder thiolatfunktionalisierte Hyaluronsäure, thiolfunktionalisierte und/oder thiolatfunktionalisierte Stärke, thiolfunktionalisiertes und/oder thiolatfunktionalisiertes Dextran, thiolfunktionalisierter und/oder thiolatfunktionalisierter Polyvinylalkohol, thiolfunktionalisiertes und/oder thiolatfunktionalisiertes Polyvinylpyrrolidon und Mischungen aus wenigstens zwei der genannten Vernetzungsmittel.

8. Zellfreie Kombination nach einem der vorhergehenden Ansprüche, **dadurch gekennzeichnet, dass** der Antikörper ausgewählt ist aus der Gruppe bestehend aus Alemtuzumab, Apolizumab, Atezolizumab, Avelumab, Bevacizumab, Blinatumomab, Catumaxomab, Cetuximab, Daratumumab, Durvalumab, Eculizumab, Elotuzumab, Emicizumab, Epratuzumab, Gemtuzumab, Gemtuzumab-Ozogamicin, Ibritumomab-Tiuxetan, Inotuzumab-Ozogamicin, Ipilimumab, Mogamulizumab, Necitumumab, Nivolumab, Obinutuzumab, Ofatumumab, Olaratumab, Oregovomab, Panitumumab, Pembrolizumab, Pertuzumab, Ramucirumab, Rituximab, Rovalpituzumab-Tesirin, Siltuximab, Tremelimumab, Tositumomab, Trastuzumab, Zanolimumab, Adalimumab, Alefacept, Anifrolumab, Basiliximab, Belimumab, Brodalumab, Canakinumab, Certolizumab, Clazakizumab, Daclizumab, Fasinumab, Guselkumab, Golimumab, Infliximab, Ixekizumab, Mavrilimumab, Muromonab-CD3, Natalizumab, Secukinumab, Sifalimumab, Sirukumab, Tocilizumab, Ustekinumab, Vedolizumab, Alirocumab, Evolocumab, Abciximab, Bezlotoxumab, Motavizumab, Palivizumab, Aducanumab, Erenumab, Ocrelizumab, Solanezumab, Ranibizumab, Dupilumab, Efalizumab, Etanercept, Nemolizumab, Ustekinumab, Tositumab, Benralizumab, Mepolizumab, Omalizumab, Reslizumab, Tralokinumab, Denosumab, Romosozumab und Mischungen aus wenigstens zwei der genannten Antikörper.

9. Zellfreie Kombination nach einem der vorhergehenden Ansprüche, **dadurch gekennzeichnet, dass** die erste Komponente und/oder die zweite Komponente in wässriger Form, vorzugsweise in Form einer wässrigen Lösung, vorliegen.

10. Zellfreie Kombination nach einem der vorhergehenden Ansprüche, **dadurch gekennzeichnet, dass** die erste Komponente und/oder die zweite Komponente als Feststoff, insbesondere als lyophilisierter Feststoff, vorliegen.

11. Zellfreie Kombination nach einem der vorhergehenden Ansprüche, **dadurch gekennzeichnet, dass** die erste Komponente und die zweite Komponente räumlich getrennt voneinander vorliegen.

12. Zellfreie Kombination nach einem der Ansprüche 1 bis 10, **dadurch gekennzeichnet, dass** die erste Komponente und die zweite Komponente miteinander vermischt sind und das vernetzbare Albumin vorzugsweise wenigstens teilweise vernetzt ist.

## Claims

1. Cell-free combination for use
- in the controlled, especially decelerated or retarded, release of active ingredient,
wherein
the cell-free combination comprises a first component and a second component, the first component comprising crosslinkable albumin and the second component comprising a crosslinking agent for the albumin, **characterized in that** the crosslinkable albumin has a degree of functionalization of 5 groups to 35 groups per albumin molecule, the crosslinking agent has a degree of functionalization of at least two groups per individual crosslinking agent molecule or, in the case of a polymeric crosslinking agent in which nonterminal monomer units and optionally also terminal monomer units are functionalized, per 100 monomer units of the polymeric crosslinking agent, the crosslinkable albumin has a fraction of 0.1 wt% to 11 wt%, based on the total volume of the cell-free combination, and the crosslinking agent has a fraction of 0.1 wt% to 30 wt%, based on the total volume of the cell-free combination, and the first component and/or the second component further comprise an active ingredient, the active ingredient being an antibody, a Fab fragment of an antibody, and/or a chondroprotective agent.

2. Cell-free combination according to Claim 1, **characterized in that** the combination is prepared or used for subcutaneous administration, preferably injection.

3. Cell-free combination according to Claim 1, **characterized in that** the combination is prepared or used for surgical, especially minimal invasive, administration, preferably injection, especially into a joint, preferably knee joint, or into a tissue cavity, especially into a tissue cavity resulting from tumor removal.

4. Cell-free combination according to any of the preceding claims, **characterized in that** the albumin is functionalized with groups, especially electrophilic groups, which are selected from the group consisting of maleimide groups, vinyl sulfone groups, acrylate groups, alkyl halide groups, azirine groups, pyridyl groups, thionitrobenzene acid groups, arylating groups, and combinations of at least two of the stated groups.

5. Cell-free combination according to any of the preceding claims, **characterized in that** the crosslinkable albumin is maleimide-functionalized albumin, especially maleimide-functionalized serum albumin, preferably of human origin.

6. Cell-free combination according to any of the preceding claims, **characterized in that** the crosslinking agent has nucleophilic groups, preferably thiol and/or thiolate groups.

7. Cell-free combination according to any of the preceding claims, **characterized in that** the crosslinking agent is selected from the group consisting of thiol-functionalized and/or thiolate-functionalized polyethylene glycol, such as dithio-polyethylene glycol, thiol-functionalized and/or thiolate-functionalized hyaluronic acid, thiol-functionalized and/or thiolate-functionalized starch, thiol-functionalized and/or thiolate-functionalized dextran, thiol-functionalized and/or thiolate-functionalized polyvinyl alcohol, thiol-functionalized and/or thiolate-functionalized polyvinylpyrrolidone, and mixtures of at least two of the stated crosslinking agents.

8. Cell-free combination according to any of the preceding claims, **characterized in that** the antibody is selected from the group consisting of alemtuzumab, apolizumab, atezolizumab, avelumab, bevacizumab, blinatumomab, catumaxomab, cetuximab, daratumumab, durvalumab, eculizumab, elotuzumab, emicizumab, epratuzumab, gemtuzumab, gemtuzumab ozogamicin, ibritumomab tiuxetan, inotuzumab ozogamicin, ipilimumab, mogamulizumab, necitumumab, nivolumab, obinutuzumab, ofatumumab, olaratumab, oregovomab, panitumumab, pembrolizumab, pertuzumab, ramucirumab, rituximab, rovalpituzumab tesirin, siltuximab, tremelimumab, tositumomab, trastuzumab, zanolimumab, adalimumab, alefacept, anifrolumab, basiliximab, belimumab, brodalumab, canakinumab, certolizumab, clazakizumab, daclizumab, fasinumab, guselkumab, golimumab, infliximab, ixekizumab, mavrilimumab, muromonab-CD3, natalizumab, secukinumab, sifalimumab, sirukumab, tocilizumab, ustekinumab, vedolizumab, alirocumab, evolocumab, abciximab, bezlotoxumab, motavizumab, palivizumab, aducanumab, erenumab, ocrelizumab, solanezumab, ranibizumab, dupilumab, efalizumab, etanercept, nemolizumab, ustekinumab, tositumab, benralizumab, mepolizumab, omalizumab, reslizumab, tralokinumab, denosumab, romosozumab, and mixtures of at least two of the stated antibodies.

9. Cell-free combination according to any of the preceding claims, **characterized in that** the first component and/or the second component are present in aqueous form, preferably in the form of an aqueous solution.

10. Cell-free combination according to any of the preceding claims, **characterized in that** the first component and/or the second component are present as a solid, especially as a lyophilized solid.

11. Cell-free combination according to any of the preceding claims, **characterized in that** the first component and the second component are present spatially separate from one another.

12. Cell-free combination according to any of Claims 1 to 10, **characterized in that** the first component and the second component are in a mixture with one another and the crosslinkable albumin is preferably at least partially crosslinked.

## Revendications

1. Association acellulaire destinée à l'utilisation
- dans la libération programmée, en particulier ralentie ou retardée, de substance active,
dans laquelle
l'association acellulaire comporte un premier composant et un second composant, le premier composant contenant de l'albumine réticulable et le second composant contenant un agent de réticulation pour l'albumine, **caractérisée en ce que** l'albumine réticulable présente un degré de fonctionnalisation de 5 groupes à 35 groupes par molécule d'albumine, l'agent de réticulation présente un degré de fonctionnalisation d'au moins deux groupes par molécule d'agent de réticulation individuelle ou dans le cas d'un agent de réticulation polymère dans lequel sont fonctionnalisées des unités monomères non en bout de chaîne et en option également des unités monomères en bout de chaîne, pour 100 unités monomères de l'agent de réticulation polymère, l'albumine réticulable présente une concentration de 0,1 % en poids à 11 % en poids, par rapport au volume total de l'association acellulaire, et l'agent de réticulation présente une concentration de 0,1 % en poids à 30 % en poids, par rapport au volume total de l'association acellulaire, et le premier composant et/ou le second composant contient/contiennent en outre une substance active, la substance active consistant en un anticorps, en un fragment Fab d'un anticorps et/ou en un chondroprotecteur.

2. Association acellulaire selon la revendication 1, **caractérisée en ce que** l'association est conçue ou utilisée pour une administration, de préférence une injection, sous-cutanée.

3. Association acellulaire selon la revendication 1, **caractérisée en ce que** l'association est conçue ou utilisée pour une administration, de préférence une injection, chirurgicale, en particulier mini-invasive, en particulier dans une articulation, de préférence articulation du genou, ou dans une cavité tissulaire, en particulier dans une cavité tissulaire résultant de l'ablation d'une tumeur.

4. Association acellulaire selon l'une quelconque des revendications précédentes, **caractérisée en ce que** l'albumine est fonctionnalisée avec des groupes, en particulier des groupes électrophiles, qui sont choisis dans le groupe constitué par les groupes maléimido, les groupes vinylsulfone, les groupes acrylate, les groupes halogénure d'alkyle, les groupes azirine, les groupes pyridyle, les groupes acide thionitrobenzoïque, les groupes arylants et des associations d'au moins des groupes cités.

5. Association acellulaire selon l'une quelconque des revendications précédentes, **caractérisée en ce que** pour ce qui est de l'albumine réticulable, il s'agit d'albumine fonctionnalisée avec un maléimide, en particulier d'albumine sérique, de préférence d'origine humaine, fonctionnalisée avec un maléimide.

6. Association acellulaire selon l'une quelconque des revendications précédentes, **caractérisée en ce que** l'agent de réticulation comporte des groupes nucléophiles, de préférence des groupes thiol et/ou thiolate.

7. Association acellulaire selon l'une quelconque des revendications précédentes, **caractérisée en ce que** l'agent de réticulation est choisi dans le groupe constitué par un polyéthylèneglycol fonctionnalisé avec un thiol et/ou fonctionnalisé avec un thiolate, tel que le dithiopolyéthylèneglycol, un acide hyaluronique fonctionnalisé avec un thiol et/ou fonctionnalisé avec un thiolate, un amidon fonctionnalisé avec un thiol et/ou fonctionnalisé avec un thiolate, un dextrane fonctionnalisé avec un thiol et/ou fonctionnalisé avec un thiolate, un poly(alcool vinylique) fonctionnalisé avec un thiol et/ou fonctionnalisé avec un thiolate, une polyvinylpyrrolidone fonctionnalisée avec un thiol et/ou fonctionnalisée avec un thiolate et des mélanges d'au moins deux des agents de réticulation cités.

8. Association acellulaire selon l'une quelconque des revendications précédentes, **caractérisée en ce que** l'anticorps est choisi dans le groupe constitué par l'alemtuzumab, l'apolizumab, l'atézolizumab, l'avélumab, le bévacizumab, le blinatumomab, le catumaxomab, le cétuximab, le daratumumab, le durvalumab, l'éculizumab, l'élotuzumab, l'émicizumab, l'épratuzumab, le gemtuzumab, le gemtuzumab-ozogamicine, l'ibritumomab-tiuxétan, l'inotuzumab-ozogamicine, l'ipilimumab, le mogamulizumab, le nécitumumab, le nivolumab, l'obinutuzumab, l'ofatumumab, l'olaratumab, l'orégovomab, le panitumumab, le pembrolizumab, le pertuzumab, le ramucirumab, le rituximab, le rovalpituzumab-tésirine, le siltuximab, le trémélimumab, le tositumomab, le trastuzumab, le zanolimumab, l'adalimumab, l'aléfacept, l'anifrolumab, le basiliximab, le bélimumab, le brodalumab, le canakinumab, le certolizumab, le clazakizumab, le daclizumab, le fasinumab, le guselkumab, le golimumab, l'infliximab, l'ixékizumab, le mavrilimumab, le muromonab-CD3, le natalizumab, le sécukinumab, le sifalimumab, le sirukumab, le tocilizumab, l'ustékinumab, le védolizumab, l'alirocumab, l'évolocumab, l'abciximab, le bezlotoxumab, le motavizumab, le palivizumab, l'aducanumab, l'érénumab, l'ocrélizumab, le solanézumab, le ranibizumab, le dupilumab, l'éfalizumab, l'étanercept, le némolizumab, l'ustékinumab, le tositumab, le benralizumab, le mépolizumab, l'omalizumab, le reslizumab, le tralokinumab, le denosumab, le romosozumab et des mélanges d'au moins deux des anticorps cités.

9. Association acellulaire selon l'une quelconque des revendications précédentes, **caractérisée en ce que** le premier composant et/ou le second composant se trouve(nt) sous forme aqueuse, de préférence sous forme d'une solution aqueuse.

10. Association acellulaire selon l'une quelconque des revendications précédentes, **caractérisée en ce que** le premier composant et/ou le second composant se trouve(nt) sous forme de solide, en particulier sous forme de solide lyophilisé.

11. Association acellulaire selon l'une quelconque des revendications précédentes, **caractérisée en ce que** le premier composant et le second composant se trouvent séparés spatialement l'un de l'autre.

12. Association acellulaire selon l'une quelconque des revendications 1 à 10, **caractérisée en ce que** le premier composant et le second composant sont mélangés l'un avec l'autre et l'albumine réticulable est de préférence au moins en partie réticulée.
